(19) Europäisches Patentamt
European Patent Office
Office européen des brevets

(11) Veröffentlichungsnummer: **0 100 899**
**B1**

(12) # EUROPÄISCHE PATENTSCHRIFT

(45) Veröffentlichungstag der Patentschrift: **15.06.88**

(21) Anmeldenummer: **83106874.7**

(22) Anmeldetag: **12.07.83**

(51) Int. Cl.⁴: **C 07 D 421/04,**
C 07 D 517/04, A 61 K 31/44,
A 61 K 31/425,
A 61 K 31/505, A 61 K 31/41

(54) **Neue Benzisoselenazolone, Verfahren zu ihrer Herstellung und diese enthaltende Pharmazeutische Präparate.**

(30) Priorität: **14.07.82 DE 3226284**

(43) Veröffentlichungstag der Anmeldung:
**22.02.84 Patentblatt 84/08**

(45) Bekanntmachung des Hinweises auf die Patenterteilung:
**15.06.88 Patentblatt 88/24**

(84) Benannte Vertragsstaaten:
**AT BE CH DE FR GB IT LI LU NL SE**

(56) Entgegenhaltungen:
**EP-A-0 044 453**
**EP-A-0 044 971**
**DE-A-3 027 074**
**ARZNEIMITTELFORSCHUNG, Band 54, Nr. 12, Dezember 1964, Seiten 1301-1306, Editio Cantor, Aulendorf, DE; R. FISCHER et al.: "On benzisothiazolones: A series with a wide range of bacteriostatic and fungistatic activity"**

**Die Akte enthält technische Angaben, die nach dem Eingang der Anmeldung eingereicht wurden und die nicht in dieser Patentschrift enthalten sind.**

(73) Patentinhaber: **A. Nattermann & Cie. GmbH**
**Nattermannallee 1**
**D-5000 Köln 30 (DE)**

(72) Erfinder: **Welter, André, Dr.**
**Reiherweg 11A**
**D-5024 Pulheim (DE)**
Erfinder: **Leyck, Sigurd, Dr.**
**Am Quechenhauf 21**
**D-5024 Pulheim 2 (DE)**
Erfinder: **Etschenberg, Eugen, Dr.**
**Hirseweg 10**
**D-5000 Köln 41 (DE)**

(74) Vertreter: **Redies, Bernd, Dr. rer. nat.**
**COHAUSZ & FLORACK Patentanwaltsbüro**
**Schumannstrasse 97 Postfach 14 01 47**
**D-4000 Düsseldorf 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 100 899 B1

**Beschreibung**

Die vorliegende Erfindung betrifft neue Benzisoselenazolone, Verfahren zu ihrer Herstellung sowie ihre Verwendung als Wirkstoff in Arzneimitteln zur Behandlung von entzündlichen Erkrankungen des rheumatischen Formenkreises.

Benzisoselenazolonderivate mit anti-arteriosklerotischen und anti-inflammatorischen Eigenschaften sind in DE—A 3027074, EP—A 44971 und EP—A 44453 beschrieben. Es handelt sich dabei um Derivate, die am Stickstoff durch Alkyl, Phenyl oder Phenylalkyl substituiert sind, wobei der Phenylrest oder das Benzisoselenazolon-Grundsystem selbst zusätzliche Substituenten aufweisen können.

Die erfindungsgemäßen Verbindungen entsprechen der allgemeinen Formel I

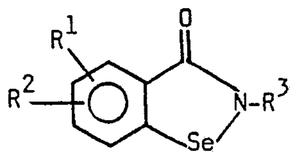

I

worin $R^1$ und $R^2$ gleich oder verschieden sein können und unabhängig voneinander Wasserstoff, Halogen, $C_1$—$C_4$-Alkyl, $C_1$—$C_4$-Alkoxy, Hydroxy, Trifluormethyl, Nitro, Di-($C_1$—$C_4$-alkyl)-amino oder $R^1$ und $R^2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Isothiazole, Pyridine bedeutet, wobei der heterocyclische Rest ein- bzw. zweifach gleich oder verschieden substituiert sein kann durch Halogen, $C_1$—$C_2$-Alkyl, $C_1$—$C_4$-Alkoxy, $C_1$—$C_4$-Alkylthio, Hydroxy, Mercapto, Trifluormethyl, Nitro, Phenyl, Nitril, Carboxy oder $C_1$—$C_4$-Alkoxycarbonyl.

Bevorzugt sind dabei Verbindungen, worin $R^1$ und $R^2$ gleich oder verschieden sein können und unabhängig voneinander Wasserstoff, Fluor, Chlor, Brom, Hydroxy, Methoxy, Trifluormethyl, Nitro oder $R_1$ und $R_2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Isothiazole, Pyridine bedeutet, wobei der heterocyclische Rest ein- bzw. zweifach gleich oder verschieden substituiert sein kann durch Fluor, Chlor, Brom, Methyl, Ethyl, Methoxy, Ethoxy, Methylthio, Ethylthio, Hydroxy, Mercapto, Trifluormethyl, Nitro, Phenyl, Nitril, Carboxy oder Methoxycarbonyl, Ethoxycarbonyl.

Besonders bevorzugt sind Verbindungen, worin $R^1$ und $R^2$ gleich oder verschieden sein können und unabhängig voneinander Wasserstoff, Chlor, Methyl, Methoxy, Nitro oder $R^1$ und $R^2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Pyridine bedeutet, wobei der heterocyclische Rest ein- oder zweifach gleich oder verschieden substituiert sein kann durch Fluor, Chlor, Brom, Methyl, Ethoxy, Methylthio, Hydroxy, Mercapto, Nitril, Nitro, Phenyl, Carboxy oder Methoxycarbonyl, Ethoxycarbonyl.

Erfindungsgemäße Verbindungen sind beispielsweise:

2-(2-Pyridyl)-1,2-benzisoselenazol-3(2H)-on,
2-(3-Pyridyl)-1,2-benzisoselenazol-3(2H)-on,
2-(4-Pyridyl)-1,2-benzisoselenazol-3(2H)-on,
6-Methyl-2-(2-pyridyl)-1,2-benzisoselenazol-3(2H)-on,
6-Chlor-2-(2-pyridyl)-1,2-benzisoselenazol-3(2H)-on,
6-Methoxy-2-(2-pyridyl)-1,2-benzisoselenazol-3(2H)-on,
5-Nitro-2-(2-pyridyl)-1,2-benzisoselenazol-3(2H)-on,
5-Chlor-2-(2-pyridyl)-1,2-benzisoselenazol-3(2H)-on,
7-Methoxy-2-(2-pyridyl)-1,2-benzisoselenazol-3(2H)-on,
6,7-Methylendioxy-2-(2-pyridyl)-1,2-benzisoselenazol-3(2H)-on,
2-(2-Chlor-3-pyridyl)-1,2-benzisoselenazol-3(2H)-on,
2-(3-Hydroxy-2-pyridyl)-1,2-benzisoselenazol-3(2H)-on,
2-(6-Hydroxy-2-pyridyl)-1,2-benzisoselenazol-3(2H)-on,
2-(6-Methoxy-3-pyridyl)-1,2-benzisoselenazol-3(2H)-on,
2-(3-Nitro-2-pyridyl)-1,2-benzisoselenazol-3(2H)-on,
2-(5-Nitro-2-pyridyl)-1,2-benzisoselenazol-3(2H)-on,
2-(3-Methyl-2-pyridyl)-1,2-benzisoselenazol-3(2H)-on,
2-(4-Methyl-2-pyridyl)-1,2-benzisoselenazol-3(2H)-on,
2-(5-Methyl-2-pyridyl)-1,2-benzisoselenazol-3(2H)-on,
2-(6-Methyl-2-pyridyl)-1,2-benzisoselenazol-3(2H)-on,
2-(3,5-Dichlor-2-pyridyl)-1,2-benzisoselenazol-3(2H)-on,
2-(4,6-Dimethyl-2-pyridyl)-1,2-benzisoselenazol-3(2H)-on,
2-(2,6-Dichlor-3-pyridyl)-1,2-benzisoselenazol-3(2H)-on,
2-(5-Chlor-2-pyridyl)-1,2-benzisoselenazol-3(2H)-on,
2-(4-Chlor-2-pyridyl)-1,2-benzisoselenazol-3(2H)-on,

2-(4-Carboxy-5-chlor-2-pyridyl)-1,2-benzisoselenazol-3(2H)-on,
2-(2-Thiazolyl)-1,2-benzisoselenazol-3(2H)-on,
6-Methyl-2-(2-thiazolyl)-1,2-benzisoselenazol-3(2H)-on,
6-Chlor-2-(2-thiazolyl)-1,2-benzisoselenazol-3(2H)-on,
6-Methoxy-(2-thiazolyl)-1,2-benzisoselenazol-3(2H)-on,
5-Nitro-2-(2-thiazolyl)-1,2-benzisoselenazol-3(2H)-on,
5-Chlor-2-(2-thiazolyl)-1,2-benzisoselenazol-3(2H)-on,
7-Methoxy-2-(2-thiazolyl)-1,2-benzisoselenazol-3(2H)-on,
6,7-Methylendioxy-2-(2-thiazolyl)-1,2-benzisoselenazol-3(2H)-on,
2-(4-Methyl-2-thiazolyl)-1,2-benzisoselenazol-3(2H)-on,
2-(5-Nitro-2-thiazolyl)-1,2-benzisoselenazol-3(2H)-on,
2-(2-Thiazolyl)-1,2-benzisoselenazol-3(2H)-on,
2-(5-Chlor-2-thiazolyl)-1,2-benzisoselenazol-3(2H)-on,
2-(3-Methyl-5-isothiazolyl)-1,2-benzisoselenazol-3(2H)-on,

Die erfindungsgemäßen Benzisoselenazolone der Formel 1 können zur Behandlung zahlreicher Krankheiten verwendet werden, wie z.B. zur Prophylaxe und Therapie von Infektionskrankneiten, zur Stimulierung des Immunsystems oder bei Selenmargelkrankheiten, wie sie bei W. Kraus und P. Oehme, Das Deut. Gesundheitswesen, 1979, 34 (37), 1713—1718 und 1979, 34 (37), 1769—1773, definiert werden.

Die Benzisoselenazolone der Formel I zeichnen sich jedoch besonders durch antiarteriosklerotische und entzündungshemmende Eigenschaften aus. Sie eignen sich besonders zur Therapie von rheumatischen Krankheiten, wie z.B. Arthrosen oder chronischer Polyarthritis, wobei die neuen Verbindungen sich durch eine sehr gute Verträglichkeit auszeichnen, da sie untoxisch sind und, im Gegensazt zu bekannten, entzündungshemmenden Therapeutika, keinerlei Ulcusbildung oder gastrointestinale Irritationen zeigen.

Die neuen Benzisoselenazolone der allgemeinen Formel I können in an sich bekannter Weise nach dem Verfahren gemäß Anspruch 14 erhalten werden. Dabie wird ein o-Chlorselenobenzoylchlorid der Formel II

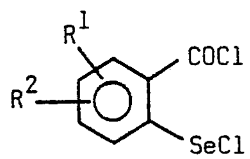

II

wobei $R^1$ und $R^2$ die in Formel I angegebene Bedeutung haben, mit einem heterocyclischen Amin der Formel III

$$R^3—NH_2 \qquad III$$

wobie $R^3$ die in Formel I angegebene Bedeutung hat, unter Ringschlußbedingungen zu dem Benzisoselenazolonen der Formel I umsetzt.

Die Herstellung der entsprechenden o-Chlorselenobenzoylchloride erfolgt nach dem Verfahren von A. Ruwet und M. Renson, Bull. Soc. Chim. Belg. 1966, *75*, 157—163.

Als Ausgansverbindungen der Formel II kommen zum Beispiel die folgenden Verbindungen in Betracht:

2-Chlorselenobenzoylchlorid,
2-Chlorseleno-4-chlorbenzoylchlorid,
2-Chlorseleno-4-fluorbenzoylchlorid,
2-Chlorseleno-4-methylbenzoylchlorid,
2-Chlorseleno-4-methoxybenzoylchlorid,
2-Chlorseleno-5-chlorbenzoylchlorid,
2-Chlorseleno-5-methoxybenzoylchlorid,
2-Chlorseleno-5-nitrobenzoylchlorid,
2-Chlorseleno-3-methoxybenzoylchlorid,
2-Chlorseleno-3,4-methylendioxybenzoylchlorid.

Als Ausgangsverbindungen III kommen z.B. in Frage:

2-Amino-3-hydroxypyridin,
2-Amino-6-hydroxypyridin,
5-Amino-2-methoxypyridin,
2-Amino-3-nitropyridin,
2-Amino-5-nitropyridin,
2-Amino-3-methylpyridin,
2-Amino-4-methylpyridin,
2-Amino-5-methylpyridin,
2-Amino-6-methylpyridin,
2-Aminopyridin,

3-Aminopyridin,
4-Aminopyridin,
2-Amino-3,5-dichloropyridin,
2-Amino-4,6-dimethylpyridin,
3-Amino-2-chlorpyridin,
2-Amino-5-chlorpyridin,
2-Amino-4-chlorpyridin,
3-Amino-2,6-dichlorpyridin,
2-Amino-4-carboxy-5-chlorpyridin,
2-Aminotetrahydropyridin,
2-Amino-3-carboxypyridin,
2-Aminothiazol,
2-Amino-4-methylthiazol,
2-Amino-5-nitrothiazol,
2-Amino-5-chlorthiazol,
5-Amino-3-methylisothiazol.

Die erfindungsgemäßen Benzisoselenazolone der allgemeinen Formel I können in üblicher Weise zu pharmazeutischen Präparaten verarbeitet werden. Zur Herstellung der pharmazeutischen Präparate, die als wirksame Komponente Benzisoselenazolone der Formel I enthalten, kann der Wirkstoff als solcher oder in Kombination mit geeigneten pharmazeutischen Verdünnungsmitteln und/oder Trägerstoffen eingesetzt werden und auf übliche Weise formuliert werden.

Im human- und veterinärmedizinischen Bereich kann der Wirkstoff in jeder beliebigen Form, z.B. systemisch, angewendet werden, unter der Vorausssetzung, daß die Ausbildung bzw. Aufrechterhaltung von ausreichenden Blut- oder Gewebespiegeln an Wirkstoff gewährleistet ist. Dies kann in oraler oder rektal oder parenteraler Gabe in geeigneten Dosen erzielt werden. Vorteilhafterweise liegt die pharmazeutische Zubereitung des Wirkstoffes in Form von Einzeldosen vor, die auf die gewünschte Verabreichung abgestimmt Sind, wie z.B. Tabletten, Dragées, Kapseln, Suppositorien, Granulate, Lösungen, Emulsionen, Suspensionen, Sole oder Gele. Die Dosierung der Verbindungen liegt üblicherweise zwischen 10—1000 mg pro Tag, vorzugsweise zwischen 30—300 mg, und kann ein- oder mehrmals, bevorzugt zwei- bis dreimal täglich, verabreicht werden.

Geeignete Trägerstoffe, die zur Herstellung oral verabreichbarer Mittel dienen, beispielsweise in Form von Tabletten, Kapseln, Granulat- oder Pulverform, sind beispielsweise Calciumcarbonat, Calciumphosphat, Stärke, Zucker, Lactose, Talkum, Magnesiumstearat, Gelatine, Polyvinylpyrrolidon, Gummi-Arabicum, Sorbit, mikrokristalline Cellulose, Polyethylenglycol, Carboxymethylcellulose, Schellack und dergleichen. Die Tabletten können auf übliche Weise überzogen werden. Flüssige Formulierungen zur oralen Verabreichung können in Form wäßriger oder öliger Suspensionen oder Lösungen, in Form eines Sirups, eines Elixiers udgl. vorliegen. Diese werden auf übliche Weise hergestellt. Bei den injizierbaren Formulierungen kann es sich um wäßrige oder ölige Suspensionen oder Lösungen, um pulvrige Zusammensetzungen mit einem Füllstoff und um gefriergetrocknete Präparationen, die vor ihrer Anwendung aufgelöst werden, udgl. handln. Diese Formulierungen werden auf übliche Weise hergestellt. Die erfindungsgemäßen pharmazeutischen Präparate können auch in Form von Suppositorien zur rektalen Verabreichung vorliegen, welche pharmazeutisch verträgliche Träger enthalten können, die als solche bekannt sind, beispielsweise Polyethylenglycol, Lanolin, Kakaobutter, Witepsol®, etc. Externe Präparationen werden vorzugsweise in Form von Salben oder Cremes hergestellt, die auf übliche Weise unter Verwendung üblicher Bestandteile hergestellt werden.

Die Herstellung der erfindungsgemäßen Verbindungen wird durch die folgenden Beispiele näher erläutert.

Beispiel 1

2-(2-Pyridyl)-1,2-benzisoselenazol-3(2H)-on.

5,08 g (0,054 Mol) 2-Aminopyridin und 16,6 ml (0,12 Mol) Triethylamin, gelöst in 120 ml absol. Tetrahydrofuran, werden unter Rühren und Eiskuhlung (Temperatur <10°C) in einer Stickstoffatmosphäre langsam zu einer Lösung von 13,75 g (0,054 Mol) o-Chlorselenobenzoesäurechlorid in 50 ml absol. Tetrahydrofuran zugetropft. Das Lösungsmittel wird zum größten Tei im Vakuum abdestilliert und der verbleibende Rückstand in Eiswasser gegossen. Der unlösliche Teil wird abgesaugt, mit viel Wasser gewaschen und aus Dioxan umkristallisiert.

Ausbeute: 13,17 g (88,3% d. Th.), F. 237—239°C
IR (in KBr): 1620 $cm^{-1}$
MS[m/e]: 276 (100%), 196 (42,2%), 168 (30,4%), 156 (34,1%), 138 (16,4%), 78 (15,2%).

Beispiel 2

2-(3-Hydroxy-2-pyridyl)-1,2-benzisoselenazol-3(2H)-on.

Zu einer kräftig gerührten Lösung von 1,76 g (0,016 Mol) 2-Amino-3-hydroxypyridin in 40 ml Tetrachlorkohlenstoff und 50 ml trockenem Pyridin wird unter Eiskühlung (Temperatur ~5°C) in einer Stickstoffatmosphäre eine Lösung von 4,08 g (0,016 Mol) o-Chlorselenobenzoesäurechlorid in 50 ml

Tetrachlorkohlenstoff zugetropft. Die Mischung wird weitere 5 Stunden bei etwa 10°C gerührt. Nach Zugabe von Chloroform wird die Lösung in Eis-Wasser gegossen. Die organische Phase wird mehrmals mit Wasser gewaschen, getrocknet und eingeengt. Der Rückstand wird aus Ethanol umkristallisiert.

Ausbeute: 2,86 g (61,3% d. Th.), F. 238°C
IR (in KBr): 1625 $cm^{-1}$
MS [m/e]: 292 (89%), 275 (9,4%), 184 (100%), 156 (35,2%), 108 (10,9%).

Analog der Vorschrift von Beispiel 1 werden hergestellt:

Beispiel 3

2-(4-Pyridyl)-1,2-benzisoselenazol-3(2H)-on.
Ausbeute: 78% d. Th., F. 259—261°C
IR (in KBr): 1662 $cm^{-1}$
MS [m/e]: 276 (70,6%), 196 (100%), 184 (7,3%), 168 (14,3%), 156 (18%), 138 (5,8%).

Beispiel 4

2-(3-Pyridyl)-1,2-benzisoselenazol-3(2H)-on.
Ausbeute: 81% d. Th., F. 265—267°C
IR (in KBr): 1635 $cm^{-1}$
MS [m/e]: 276 (77,9%), 196 (100%), 184 (7,6%), 168 (22,8%), 156 (18,8%), 138 (7,7%).

Beispiel 5

2-(2-Chlor-3-pyridyl)-1,2-benzisoselenazol-3(2H)-on.
Ausbeute: 72% d. Th., F. 199—201°C
IR (in KBr): 1666 $cm^{-1}$
MS [m/e]: 310 (25%), 275 (100%), 247 (9,5%), 184 (11,6%), 156 (28%).

Beispiel 6

2-(2-Thiazolyl)-1,2-benzisoselenazol-3(2H)-on.
Ausbeute: 72% d. Th., F. 288—290°C
IR (in KBr): 1658 $cm^{-1}$
MS [m/e]: 282 (85,6%), 234 (12,2%), 202 (18,5%), 184 (100%), 156 (59,5%), 136 (11,6%), 117 (16%).

Beispiel 7

2-(5-Chlor-2-pyridyl)-1,2-benzisoselenazol-3(2H)-on.
Ausbeute: 54% d. Th., F. 267—269°C
IR (in KBr): 1612 $cm^{-1}$
MS [m/e]: 310 (100%), 275 (14,63%), 230 (46,80%), 202 (25,85%), 167 (7,65%), 156 (33,75).

Beispiel 8

2-(6-Methoxy-3-pyridyl)-1,2-benzisoselenazol-3(2H)-on.
Ausbeute: 45% d. Th., F. 224—225°C
IR (in KBr): 1588 $cm^{-1}$
MS [m/e]: 306 (100%), 277 (8,23%), 225 (45,19%), 197 (27,93%), 156 (14,49%), 80 (23,58%).

Beispiel 9

2-(2,6-Dichlor-3-pyridyl)-1,2-benzisoselenazol-3(2H)-on.
Ausbeute: 38% d. Th., F. 194—195°C
IR (in KBr): 1623 $cm^{-1}$
MS [m/e]: 344 (22,0%), 309 (100%), 184 (11,5%), 156 (26,3%).

Beispiel 10

2-(4,6-Dimethyl-2-pyridyl)-1,2-benzisoselenazol-3(2H)-on.
Ausbeute: 77% d. Th., F. 238—241°C
MS [m/e]: 304 (100%), 224 (64,50%), 196 (13,54%), 77 (16,10%).

Analog den Vorschriften der Beispielel-2 werden hergestellt:
2-(6-Hydroxy-2-pyridyl)-1,2-benzisoselenazol-3(2H)-on,
2-(3-Nitro-2-pyridyl)-1,2-benzisoselenazol-3(2H)-on,
2-(5-Nitro-2-pyridyl)-1,2-benzisoselenazol-3(2H)-on,
6-Methyl-2-(2-pyridyl)-1,2-benzisoselenazol-3(2H)-on,
6-Chlor-2-(2-pyridyl)-1,2-benzisoselenazol-3(2H)-on,
6-Methoxy-2-(2-pyridyl)-1,2-benzisoselenazol-3(2H)-on,
5-Nitro-2-(2-pyridyl)-1,2-benzisoselenazol-3(2H)-on,
5-Chlor-2-(2-pyridyl)-1,2-benzisoselenazol-3(2H)-on,

7-Methoxy-2-(2-pyridyl)-1,2-benzisoselenazol-3(2H)-on,
6,7-Methylendioxy-2-(2-pyridyl)-1,2-benzisoselenazol-3(2H)-on,
2-(3-Methyl-2-pyridyl)-1,2-benzisoselenazol-3(2H)-on,
2-(4-Methyl-2-pyridyl)-1,2-benzisoselenazol-3(2H)-on,
2-(5-Methyl-2-pyridyl)-1,2-benzisoselenazol-3(2H)-on,
2-(6-Methyl-2-pyridyl)-1,2-benzisoselenazol-3(2H)-on,
2-(3,5-Dichlor-2-pyridyl)-1,2-benzisoselenazol-3(2H)-on,
2-(4-Chlor-2-pyridyl)-1,2-benzisoselenazol-3(2H)-on,
2-(4-Carboxy-5-chlor-2-pyridyl)-1,2-benzisoselenazol-3(2H)-on,
6-Methyl-2-(2-thiazolyl)-1,2-benzisoselenazol-3(2H)-on,
6-Chlor-2-(2-thiazolyl)-1,2-benzisoselenazol-3(2H)-on,
6-Methoxy-2-(2-thiazolyl)-1,2-benzisoselenazol-3(2H)-on,
5-Nitro-2-(2-thiazolyl)-1,2-benzisoselenazol-3(2H)-on,
5-Chlor-2-(2-thiazolyl)-1,2-benzisoselenazol-3(2H)-on,
7-Methoxy-2-(2-thiazolyl)-1,2-benzisoselenazol-3(2H)-on,
6,7-Methylendioxy-2-(2-thiazolyl)-1,2-benzisoselenazol-3(2H)-on,
2-(4-Methyl-2-thiazolyl)-1,2-benzisoselenazol-3(2H)-on,
2-(5-Nitro-2-thiazolyl)-1,2-benzisoselenazol-3(2H)-on,
2-(2-Thiazolinyl)-1,2-benzisoselenazol-3(2H)-on,
2-(5-Chlor-2-thiazolyl)-1,2-benzisoselenazol-3(2H)-on,
2-(3-Methyl-5-isothiazolyl)-1,2-benzisoselenazol-3(2H)-on,

**Patentansprüche für die Vertragsstaaten: BE CH DE FR GB IT LI LU NL SE**

1. Benzisoselenazolone der allgemeinen Formel I

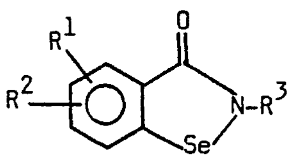

I

worin $R^1$ und $R^2$ gleich oder verschieden sein können und unabhängig voneinander Wasserstoff, Halogen, $C_1$—$C_4$-Alkyl, $C_1$—$C_4$-Alkoxy, Hydroxy, Trifluormethyl, Nitro, Di-($C_1$—$C_4$-alkyl)-amino oder $R^1$ und $R^2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Isothiazole und Pyridine bedeutet, wobei der heterocyclische Rest einfach bzw. zweifach gleich oder verschieden substituiert sein kann durch Halogen, $C_1$—$C_2$-Alkyl, $C_1$—$C_4$-Alkoxy, $C_1$—$C_4$-Alkylthio, Hydroxy, Mercapto, Trifluormethyl, Nitro, Phenyl, Nitril und Carboxy oder $C_1$—$C_4$-Alkoxycarbonyl.

2. Benzisoselenazolone gemäß Anspruch 1, worin $R^1$ und $R^2$ gleich oder verschieden sein können und unabhängig voneinander Wasserstoff, Fluor, Chlor, Brom, Hydroxy, Methoxy, Trifluormethyl, Nitro oder $R^1$ und $R^2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Isothiazole, Pyridine bedeutet, wobei der heterocyclische Rest ein- bzw. zweifach gleich oder verschieden substituiert sein kann durch Fluor, Chlor, Brom, Methyl, Ethyl, Methoxy, Ethoxy, Methylthio, Ethylthio, Hydroxy, Mercapto, Trifluormethyl, Nitro, Phenyl, Nitril, Carboxy oder Methoxycarbonyl, Ethoxycarbonyl.

3. Benzisoselenazolone gemäß Anspruch 1, worin $R^1$ und $R^2$ gleich oder verschieden sein können und unabhängig voneinander Wasserstoff, Chlor, Methyl, Methoxy, Nitro oder $R^1$ und $R^2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Pyridine bedeutet, wobei der heterocyclische Rest ein- oder zweifach gleich oder verschieden substituiert sein kann durch Fluor, Chlor, Brom, Methyl, Ethoxy, Methylthio, Hydroxy, Mercapto, Nitril, Nitro, Phenyl, Carboxy oder Methoxycarbonyl, Ethoxycarbonyl.

4. 2-(2-Pyridyl)-1,2-benzisoselenazol-3(2H)-on.

5. 2-(3-Pyridyl)-1,2-benzisoselenazol-3(2H)-on.

6. 2-(4-Pyridyl)-1,2-benzisoselenazol-3(2H)-on.

7. 2-(2-Chlor-3-pyridyl)-1,2-benzisoselenazol-3(2H)-on.

8. 2-(2-Thiazolyl)-1,2-benzisoselenazol-3(2H)-on.

9. 2-(4-Methyl-2-thiazolyl)-1,2-benzisoselenazol-3(2H)-on.

10. 2-(2,6-Dichlor-3-pyridyl)-1,2-benzisoselenazol-3(2H)-on.

11. 2-(6-Methoxy-3-pyridyl)-1,2-benzisoselenazol-3(2H)-on.

12. 2-(5-Chlor-2-pyridyl)-1,2-benzisoselenazol-3(2H)-on.

13. 2-(4,6-Dimethyl-2-pyridyl)-1,2-benzisoselenazol-3(2H)-on.

14. Verfahren zur Herstellung von Verbindungen gemäß den Ansprüchen 1—13, dadurch gekennzeichnet, daß man in an sich bekannter Weise Benzoylchloride der allgemeinen Formel II

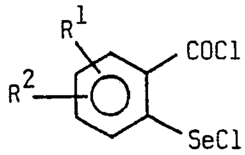

II

worin $R^1$ und $R^2$ die gleiche Bedeutung wie in Formel I haben, mit einem heterocyclischen Amin der allgemeinen Formel III

$$R^3—NH_2 \qquad III$$

worin $R^3$ die in Formel I angegebene Bedeutung hat, unter Ringschlußbedingungen zu den Benzisoselenazolen der allgemeinen Formel I umsetzt.

15. Pharmazeutische Präparate, dadurch gekennzeichnet, daß sie eine Verbindung der Formel I gemäß den Ansprüchen 1—13 als Wirkstoff im Gemisch mit üblichen pharmazeutischen Hilfs- und Trägerstoffen enthalten.

**Patentanspruch für den Vertragsstaat: AT**

Verfahren zur Herstellung von Benzisoselenazolonen der allgemeinen Formel I

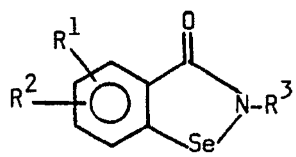

I

worin $R^1$ und $R^2$ gleich oder verschieden sein können und unabhängig voneinander Wasserstoff, Halogen, $C_1$—$C_4$-Alkyl, $C_1$—$C_4$-Alkoxy, Hydroxy, Trifluormethyl, Nitro, Di-($C_1$—$C_4$-Alkyl)-amino oder $R^1$ und $R^2$ zusammen Methylendioxy bedeuten, während $R^3$ einen heterocyclischen ungesättigten Rest mit einem bis zwei Heteroatomen der Elemente Stickstoff und/oder Schwefel aus der Gruppe der Thiazole, Isothiazole und Pyridine bedeutet, wobei der heterocyclische Rest einfach bzw. zweifach gleich oder verschieden substituiert sein kann durch Halogen, $C_1$—$C_2$-Alkyl, $C_1$—$C_4$-Alkoxy, $C_1$—$C_4$-Alkylthio, Hydroxy, Mercapto, Trifluormethyl, Nitro, Phenyl, Nitril und Carboxy oder $C_1$—$C_4$-Alkoxycarbonyl, dadurch gekennzeichnet, daß man in an sich bekannter Weise Benzoylchloride der allgemeinen Formel II

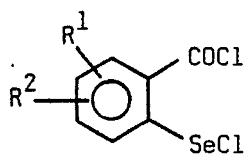

II

worin $R^1$ und $R^2$ die gleiche Bedeutung wie in Formel I haben, mit einem heterocyclischen Amin der allgemeinen Formel III

$$R^3—NH_2 \qquad III$$

worin $R^3$ die in Formel I angegebene Bedeutung hat, unter Ringschlußbedingungen zu den Benzisoselenazolen der allgemeinen Formel I umsetzt.

**Revendications pour les Etats contractants: BE CH DE FR GB IT LI LU NL SE**

1. Benzisosélénazolones de formule générale I

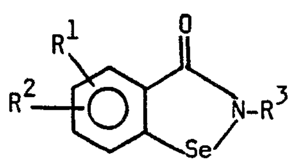

I

où $R^1$ et $R^2$ peuvent être identiques ou différents et représentent indépendamment l'un de l'autre un hydrogène, halogène, alcoyle en $C_1$—$C_4$, alcoxy en $C_1$—$C_4$, hydroxy, trifluorométhyle, nitro, di-(alcoyle en

$C_1$—$C_4$)-amino ou R' et R² représentent ensemble un méthylènedioxy, tandis que $R^3$ représente un radical non saturé hétérocyclique comportant de 1 à 2 hétéroatomes des éléments azote et/ou soufre du groupe des thiazoles, isothiazoles, et pyridines où le radical hétérocyclique peut être mono- ou disubstitué de manière identique ou différente par un halogène, alcoyle en $C_1$—$C_2$, alcoxy en $C_1$—$C_4$, alcoylthio en $C_1$—$C_4$, hydroxy, mercapto, trifluorométhyle, nitro, phényle, nitrile et carboxy, ou alcoxycarbonyle en $C_1$—$C_4$.

2. Benzisosélénazolones selon la revendication 1, où $R^1$ et $R^2$ peuvent être identiques ou différents et représentent indépendamment l'un de l'autre un hydrogène, fluor, chlore, brome, hydroxy, méthoxy, trifluorométhyle, nitro ou $R^1$ et $R^2$ représentent ensemble un méthylènedioxy, tandis que $R^3$ représente un radical non saturé hétérocyclique comportant de 1 à 2 hétéroatomes des éléments azote et/ou soufre du groupe des thiazoles, isothiazoles, pyridines, où le radical hétérocyclique peut être mono- ou disubstitué de manière identique ou différente par un fluor, chlore, bromo, méthyle, éthyle, méthoxy, éthoxy, méthylthio, éthylthio, hydroxy, mercapto, trifluorométhyle, nitro, phényle, nitrile, carboxy ou méthoxycarbonyle, éthoxycarbonyle.

3. Benzisosélénazolones selon la revendication 1, où $R^1$ et $R^2$ peuvent être identiques ou différents et représentent indépendamment l'un de l'autre un hydrogène, chlore, méthyle, méthoxy, nitro ou $R^1$ et $R^2$ représentent ensemble un méthylènedioxy, tandis que $R^3$ représente un radical non saturé hétérocyclique comportant de 1 à 2 hétéroatomes des éléments azote et/ou soufre du groupe des thiazoles, isothiazoles, pyridines, où le radical hétérocyclique peut être mono- ou disubstitué de manière identique ou différente par un fluor, chlore, brome, méthyle, méthoxy, éthoxy, méthylthio, hydroxy, mercapto, nitrile, nitro, phényle, carboxy ou méthoxycarbonyle, éthoxycarbonyle.

4. 2-(2-pyridyl)-1,2-benzisosélénazol-3(2H)-one.

5. 2-(3-pyridyl)-1,2-benzisosélénazol-3(2H)-one.

6. 2-(4-pyridyl)-1,2-benzisosélénazol-3(2H)-one.

7. 2-(2-chloro-3-pyridyl)-1,2-benzisosélénazol-3(2H)-one.

8. 2-(2-thiazolyl)-1,2-benzisoselenazol-3(2H)-one.

9. 2-(4-méthyl-2-thiazolyl)-1,2-benzisosélénazol-3(2H)-one.

10. 2-(2,6-dichloro-3-pyridyl)-1,2-benzisosélénazol-3(2H)-one.

11. 2-(6-méthoxy-3-pyridyl)-1,2-benzisosélénazol-3(2H)-one.

12. 2-(5-chloro-2-pyridyl)-1,2-benzisosélénazol-3(2H)-one.

13. 2-(4,6-diméthyl-2-pyridyl)-1,2-benzisosélénazol-3(2H)-one.

14. Procédé de préparation de composés selon les revendications 1—13, caractérisé en ce qu'on fait réagir de façon connue des chlorures de benzoyle de formulé dé générale II

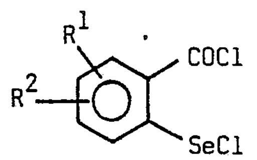

II

où $R^1$ et $R^2$ ont la même signification que dans la formule I, avec une amine hétérocyclique de formule générale III

$$R^3—NH_2 \qquad III$$

où $R^3$ a la signification donnée dans la formule I dans des conditions de cyclisation pour donner les benzisosélénazoles de formule générale I.

15. Préparations pharmaceutiques caractérisées en ce qu'elles contiennent un composé de formule I selon les revendications 1—13 comme substance active mélangée à des additifs et supports pharmaceutiques habituels.

**Revendication pour l'Etat contractant: AT**

Procédé de préparation de benzisosélénazolones de formule générale I

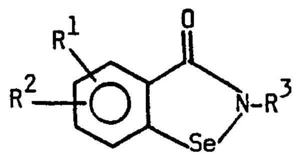

I

où $R^1$ et $R^2$ peuvent être identiques ou différents et représentent indépendamment l'un de l'autre un hydrogène, halogène, alcoyle en $C_1$—$C_4$, alcoxy en $C_1$—$C_4$, hydroxy, trifluorométhyle, nitro, di-(alcoyle en $C_1$—$C_4$)-amino ou $R^1$ et $R^2$ représentent ensemble un méthylènedioxy, tandis que $R^3$ représente un radical non saturé hétérocyclique comportant de 1 à 2 hétéroatomes des éléments azote et/ou soufre du groupe

des thiazoles, isothiazoles, et pyridines où le radical hétérocycliques peut être mono- ou disubstitué de manière identique ou différente par un halogène, alcoyle en $C_1$—$C_2$, alcoxy en $C_1$—$C_4$, alcoylthio en $C_1$—$C_4$, hydroxy, mercapto, trifluorométhyle, nitro, phényle, nitrile et carboxy, ou alcoxycarbonyle en $C_1$—$C_4$, caractérisé en ce qu'on fait réagir de façon connue des chlorures de benzoyle de formule générale II

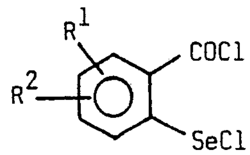

II

où $R^1$ et $R^2$ ont la même signification que dans la formule I, avec une amine hétérocyclique de formule générale III

$$R^3—NH_2 \qquad III$$

où $R^3$ a la signification donnée dans la formule I dans des conditions de cyclisation pour donner les benzisosélénazoles de formule générale I.

**Claims for the Contracting States: BE CH DE FR GB IT LI LU NL SE**

1. Benzisoselenazolones of the general formula I:

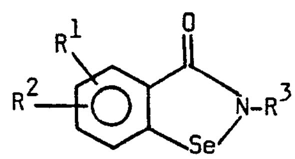

I

in which $R^1$ and $R^2$ can be identical or different and, independently of one another, denote hydrogen, halogen, $C_1$—$C_4$-alkyl, $C_1$—$C_4$-alkoxy, hydroxyl, trifluoromethyl, nitro, di-($C_1$—$C_4$-alkyl)-amino or $R^1$ and $R^2$ together denote methylenedioxy, while $R^3$ denotes a heterocyclic unsaturated radical having 1 to 2 hetero atoms of the elements nitrogen and/or sulphur from the group comprising thiazoles, isothiazoles and pyridines, it being possible for the heterocyclic radical to be substituted once or twice, identically or differently, by halogen, $C_1$—$C_2$-alkyl, $C_1$—$C_4$-alkoxy, $C_1$—$C_4$-alkylthio, hydroxyl, mercapto, trifluoromethyl, nitro, phenyl, nitrile and carboxyl or $C_1$—$C_4$-alkoxy carbonyl.

2. Benzisoselenazolones according to claim 1, in which $R^1$ and $R^2$ can be identical or different and, independently of one another, denote hydrogen, flourine, chlorine, bromine, hydroxyl, methoxy, trifluoromethyl, nitro or $R^1$ and $R^2$ together denote methylenedioxy, while $R^3$ denotes a heterocyclic unsaturated radical having 1 to 2 hetero atoms of the elements nitrogen and/or sulphur from the group comprising the thiazoles, isothiazoles, pyridines, it being possible for the heterocyclic radical to be substituted once or twice, identically or differently, by fluorine, chlorine, bromine, methyl, ethyl, methoxy, ethoxy, methylmercapto, ethylmercapto, hydroxyl, mercapto, trifluoromethyl, nitro, phenyl, nitrile, carboxyl or methoxycarbonyl and ethoxycarbonyl.

3. Benzisoselenazolones according to claim 1, in which $R^1$ and $R^2$ can be identical or different and, independently of one another, denote hydrogen, chlorine, methyl, methoxy, nitro or $R^1$ and $R^2$ together denote methylenedioxy, while $R^3$ denotes a heterocyclic unsaturated radical having 1 to 2 hetero atoms of the elements nitrogen and/or sulphur from the group comprising the thiazoles, pyridines, it being possible for the heterocyclic radical to be substituted once or twice, identically or differently, by fluorine, chlorine, bromine, methyl, methoxy, ethoxy, methylmercapto, hydroxyl, mercapto, nitrile, nitro, phenyl, carboxyl or methoxycarbonyl and ethoxycarbonyl

4. 2-(2-pyridyl)-1,2-benzisoselenazol-3(2H)-one.

5. 2-(3-pyridyl)-1,2-benzisoselenazol-3(2H)-one.

6. 2-(4-pyridyl)-1,2-benzisoselenazol-3(2H)-one.

7. 2-(2-chlor-3-pyridyl)-1,2-benzisoselenazol-3(2H)-one.

8. 2-(2-thiazolyl)-1,2-benzisoselenazol-3(2H)-one.

9. 2-(4-methyl-2-thiazolyl)-1,2-benzisoselenazol-3(2H)-one.

10. 2-(2,6-dichloro-3-pyridyl)-1,2-benzisoselenazol-3(2H)-one.

11. 2-(6-methoxy-3-pyridyl)-1,2-benzisoselenazol-3(2H)-one.

12. 2-(5-chloro-2-pyridyl)-1,2-benzisoselenazol-3(2H)-one.

13. 2-(4,6-dimethyl-2-pyridyl)-1,2-benzisoselenazol-3(2H)-one.

14. Process for the preparation of compounds according to claims 1 to 13, characterised in that benzoyl chlorides of the general formula II

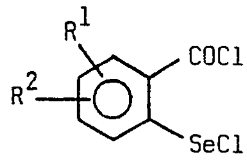

II

in which $R^1$ and $R^2$ have the same meaning as in formula I, are reacted, in a manner known per se, with a heterocyclic amine of the general formula III

$$R^3—NH_2 \qquad III$$

$R^3$ having the meaning indicated in formula I, under conditions of ring closure to give the benzisoselanazolones of the general formula I.

15. Pharmaceutical products, characterised in that they contain a compound of the formula I according to claims 1 to 13 as the active ingredient in a mixture with customary pharmaceutical auxiliaries and vehicles.

**Claim for the Contracting State: AT**

Process for the preparation of benzisoselenazolones of the general formula I

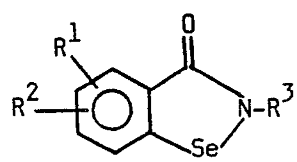

I

in which $R^1$ and $R^2$ can be identical or different and, independently of one another, denote hydrogen, halogen, $C_1—C_4$-alkyl, $C_1—C_4$-alkoxy, hydroxyl, trifluoromethyl, nitro, di-($C_1—C_4$-alkyl)-amino or $R^1$ and $R^2$ together denote methylenedioxy, while $R^3$ denotes a heterocyclic unsaturated radical having 1 to 2 hetero atoms of the elements nitrogen and/or suphur from the group comprising thiazoles, isothiazoles and pyridines, it being possible for the heterocyclic radical to be substituted once or twice, identically or differently, by halogen, $C_1—C_2$-alkyl, $C_1—C_4$-alkoxy, $C_1—C_4$-alkylthio, hydroxyl, mercapto, trifluoromethyl, nitro, phenyl, nitrile and carboxyl or $C_1—C_4$-alkoxy carbonyl, characterised in that benzoyl chlorides of the general formula II

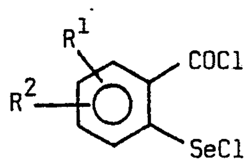

II

in which $R^1$ and $R^2$ have the same meaning as in formula I are reacted with a heterocyclic amine of the general formula III

$$R^3—NH_2 \qquad III$$

$R^3$ having the meaning indicated in formula I, under conditions of ring closure to give the benzisoselanazolones of the general formula I.